(19) Europäisches Patentamt
European Patent Office
Office européen des brevets

(11) Veröffentlichungsnummer: **0 199 945**
**A2**

(12) # EUROPÄISCHE PATENTANMELDUNG

(21) Anmeldenummer: **86103389.2**

(22) Anmeldetag: **13.03.86**

(51) Int. Cl.⁴: **C07D 207/325**, C07C 127/19, A01N 47/00

(30) Priorität: **23.03.85 DE 3510653**
**23.03.85 DE 3510652**

(43) Veröffentlichungstag der Anmeldung:
**10.12.86 Patentblatt 86/45**

(84) Benannte Vertragsstaaten:
**AT BE CH DE FR GB IT LI NL**

(71) Anmelder: **HOECHST AKTIENGESELLSCHAFT**
**Postfach 80 03 20**
**D-6230 Frankfurt am Main 80(DE)**

(72) Erfinder: **Koch, Volker, Dr.**
**Altkönigstrasse 5**
**D-6233 Kelkheim (Taunus)(DE)**
Erfinder: **Knauf, Werner, Dr.**
**Im Kirschgarten 24**
**D-6239 Eppstein/Taunus(DE)**
Erfinder: **Waltersdorfer, Anna, Dr.**
**Rauenthaler Weg 28**
**D-6000 Frankfurt am Main 71(DE)**
Erfinder: **Bonin, Werner, Dr.**
**Im Schulzehnten 18**
**D-6233 Kelkheim (Taunus)(DE)**

(54) **Neue Carbamoylarylcarboximidsäureester, Verfahren zu ihrer Herstellung, sie enthaltende Mittel und ihre Verwendung als Schädlingsbekämpfungsmittel.**

(57) Die Verbindungen der Formel I,

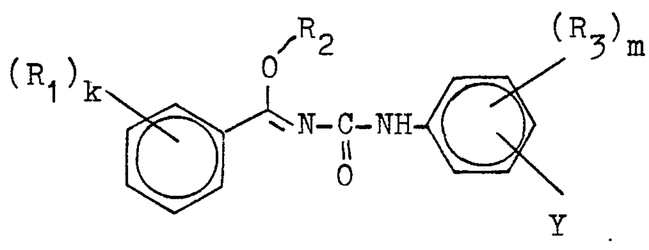

worin Y = 4-F, Pyrrolyl oder (subst.) Phenoxy, $R_1$ = Wasserstoff, Halogen, (Halogen)Alkyl, (Halogen)-Alkoxy, $R_2$ = Alkyl, Cycloalkyl, Alkenyl, Alkinyl, Benzyl, die halogeniert sein können $R_3$ Wasserstoff, Halogen, (Halogen)Alkyl, (Halogen)Alkoxy, k, m = 1 bis 3 bedeuten, besitzen gute insektizide Wirksamkeit.

EP 0 199 945 A2

# Neue Carbamoylarylcarboximidsäureester, Verfahren zu ihrer Herstellung, sie enthaltende Mittel und ihre Verwendung als Schädlingsbekämpfungsmittel

Es ist bereits bekannt, daß bestimmte N-Carbamyl-2,6-di-fluorbenz(thio)-carboximidsäureester insektizide Eigenschaften aufweisen (Europäische Patentschrift Nr. 5944). Diese besitzen jedoch Nachteile in der Anwendung wie unzureichende Wirksamkeiten.

Es wurden nun neue substituierte N-Carbamylarylcarboximidsäureester mit vorteilhaften insektiziden Eigenschaften gefunden.

Gegenstand der vorliegenden Erfindung sind daher die Verbindungen der Formel I,

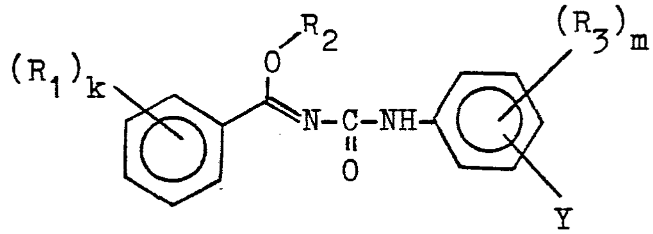

worin Y= einen Rest 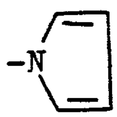 , 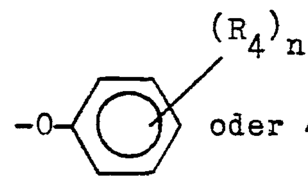 oder 4-Fluor,

$R_1$ = Wasserstoff, Halogen, $(C_1\text{-}C_4)$Alkyl, $(C_1\text{-}C_4)$-Alkoxy, die beide ein-oder mehrfach durch Halogen substituiert sein können,

$R_2$ = $(C_1\text{-}C_6)$Alkyl, $C_3\text{-}C_6)$Cycloalkyl, $(C_3\text{-}C_6)$Alkenyl, $(C_3\text{-}C_6)$Alkinyl oder Benzyl, wobei alle diese Reste ein-oder mehrfach durch Halogen substituiert sein können,

$R_3$, $R_4$ = unabhängig voneinander Wasserstoff, Halogen, $(C_1\text{-}C_6)$Alkyl, $(C_1\text{-}C_6)$Alkoxy, die beide ein-oder mehrfach durch Halogen substituiert sein können,

k, m, n = unabhängig voneinander eine ganze Zahl von 1 bis 3 bedeuten, mit der Maßgabe, daß wenn Y=3-Fluor bedeutet, $(R_3)_m$ für 3,5-Dichlor-2-fluor und $(R_1)_k$ für 2,6-Difluor stehen muß.

Im Falle der Mehrfachsubstitution können die Reste $R_1$, $R^3$, $R^4$ gleiche oder verschiedene Bedeutungen besitzen.

Bevorzugt sind Verbindungen der Formel I mit $R_2$ = $(C_1\text{-}C_6)$-Alkyl.

Gegenstand der vorliegenden Erfindung sind auch Verfahren zur Herstellung der Verbindungen der Formel (I), dadurch gekennzeichnet, daß man

a) einen substituierten Arylcarboximidsäureester der Formel (II)

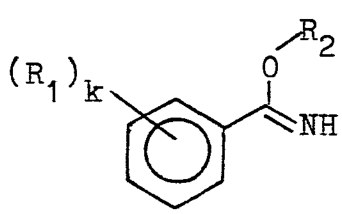

(II)

mit einer Verbindung der Formel (III)

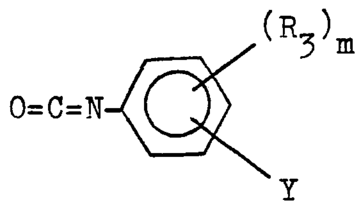

(III)

oder mit einer Verbindung der Formel (IV)

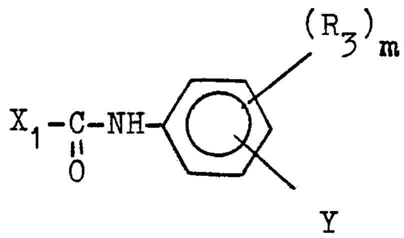

(IV)

worin $X_1$ eine nucleofuge Abgangsgruppe außer Halogen wie beispielsweise $(C_1-C_6)$-Alkoxy, Phenoxy, die halo geniert sein können, Imidazol oder Triazol bedeutet, oder

b) einen substituierten Arylcarboximidsäureester-Derivat der Formel (V)

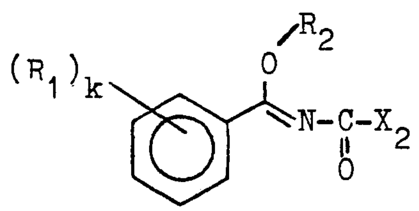

(V)

worin $X_2$ die Bedeutung von $X_1$ besitzt und zusätzlich für Halogen steht,

mit einem substituierten Anilin der Formel - (VI)

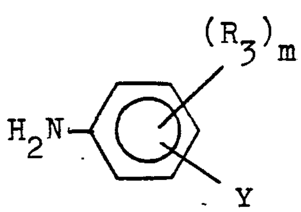

(VI)

oder

c) ein substituiertes 1,3,5-Oxadiazin der Formel (VII)

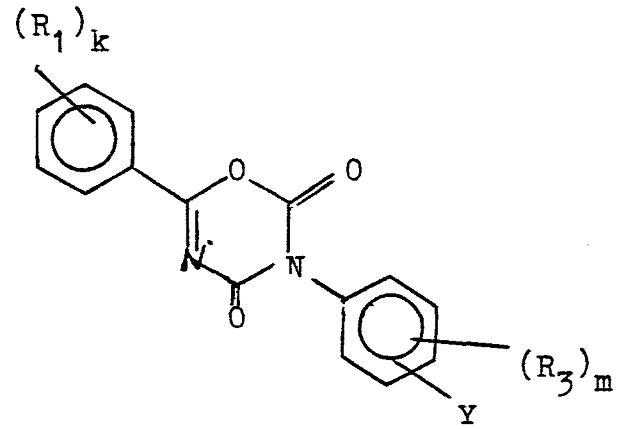

(VII)

mit einem Alkohol der Formel (VIII)

$R_2$-OH (VIII)

umsetzt.

Die Als Ausgangsverbindungen beim Herstellungsverfahren a) zu verwendenden Arylcarboximidsäureester der Formel (II) sind zum Teil neu und können nach an sich literaturbekannten Verfahren hergestellt werden.

1. durch sauer oder basisch katalysierte Addition von Alkoholen der Formel (VIII) an die substituierten Nitrile der Formel (IX)

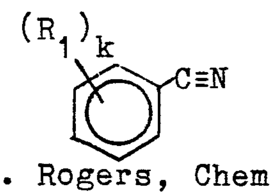

(IX)

(s.R. Rogers, Chem. Rev. 61, 179 (1961))

2. durch Umesterung von leicht zugänglichen Benzcarboximidsäureestern der Formel (II) mit Verbindungen der Formel (VIII)

(s. R.J. Kauffmann, J. Am. Chem. Soc. 45, 1744 (1923)) oder

3. durch O-Alkylierung von substituierten Benzamiden der Formel (X)

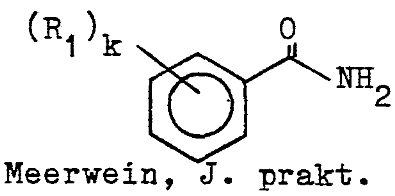

(X)

(s.H. Meerwein, J. prakt. Chem. 15, 154 (1940)).

Die als Ausgangsverbindungen beim Herstellungsverfahren a) zu verwendenden Verbindungen der Formel (III) und (IV) sind entweder bekannt oder können analog nach literaturbekannten Verfahren hergestellt werden (Houben-Weyl, Band VIII, 1952, Houben-Weyl, Band E 4, 1983).

Die als Ausgangsverbindungen beim Herstellungsverfahren b) zu verwendenden substituierten Arylcarboximidoester-Derivate der Formel (V) sind in an sich bekannter Weise aus Verbindungen der Formel (II) zugänglich (Houben-Weyl, Band E 4, 1983). Die für Weg b) benötigten Aniline der Formel (VI) sind entweder bekannt oder können nach an sich literaturbekannten Verfahren hergestellt werden (Houben-Weyl, Bank XI/1 (1957)).

Die als Ausgangsverbindungen beim Herstellungsverfahren c) zu verwendenden 1,3,5-Oxadiazin-Derivate der Formel (VII) sind nach an sich literaturbekannten Verfahren erhältlich (US-PS 4,150,158).

Die genannte Verfahrensvariante a) zur Herstellung von N-Carbamylarylbenzcarboximidsäureestern der Formel (I) wird mit oder ohne Verdünnungsmittel durchgeführt. Als Verdünnungsmittel eignen sich praktisch alle aprotischen Lösungsmittel. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glycoldimethylether und Diglycoldimethylether, Tetrahydrofuran und Dioxan, Ketone, wie Aceton, Methylethyl-, Methylisopropyl-und Methylisobutylketon, Ester, wie Essigsäuremethylester und -ethylester, Nitrile, wie z.B. Acetonitril und Propionitril, Amide, wie z.B. Dimethylformamid, Dimethylacetamid und N-Methyl-pyrrolidon, sowie Dimethylsulfoxid, Tetramethylensulfon und Hexamethylphosphorsäuretriamid.

Die Reaktionstemperatur liegt im allgemeinen zwischen -10°C und +150°C, vorzugsweise zwischen +10°C und 105°C.

Die Ausgangsstoffe werden gewöhnlich in äquimolarem Mengen eingesetzt. Ein Überschuß der einen oder anderen Reaktionskomponente ist jedoch möglich.

Bei der Verfahrensvariante b) wird die Umsetzung vorzugsweise in einem Verdünnungsmittel vorgenommen. Geeignet sind alle neutralen, nicht nucleophilen organischen Lösungsmittel, bevorzugt jedoch die beim Verfahren a) genannten.

Die Reaktionstemperatur liegt im allgemeinen zwischen -10°C und +140°C, vorzugsweise zwischen +10°C und +80°C.

Die Ausgangsstoffe werden wie beim Verfahren a) gewöhnlich in äquimolaren Mengen eingesetzt. Ein Überschuß der einen oder anderen Reaktionskomponente ist jedoch möglich.

Bei der Herstellung der Verbindungen der Formel (I) nach der Verfahrensvariante c) arbeitet man bei der Umsetzung der Verbindungen der Formel - (VII) mit Komponenten (VIII) zweckmäßigerweise in einem Überschuß der Komponente (VIII).

Die Reaktionstemperatur liegt im allgemeinen zwischen +20°C und +150°C, vorzugsweise zwischen +50°C und +100°C.

Die Isolierung und gegebenenfalls Reinigung der Verbindungen der Formel (I) erfolgt nach allgemein üblichen Methoden, z.B. durch Abdampfen des Lösungsmittels (gegebenenfalls unter vermindertem Druck) und anschließendes Umkristallisieren des Rückstands oder durch Chromatographie.

Die Verbindungen der Formel (I) werden dabei als E-oder Z-Isomere oder als E/Z-Gemische erhalten. Die E/Z-Gemische lassen sich durch an sich bekannte physikalische Prozesse, wie fraktionierte Kristallisation, trennen. Beide Isomere sind biologisch aktiv und werden daher beide von vorliegender Erfindung umfaßt.

Die Verbindungen der Formel (I) sind in den meisten organischen Lösungsmitteln gut löslich.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, ganz besonders bevorzugt zur Bekämpfung von Insekten, die in der Landwirtschaft, in Forsten, im Vorrats-und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blatella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp., Aus der Ordnung der Anoplura z.B. Phylloera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Bravicornyne brassicae, Cryptomyzus ribis, Doralis fabae, Doralis pomi, Eriosoma lanigerum, Hyalopterus arundinis, Macrosiphium avenae, Myzus spp., Phorodon humuli, Rhopalosiphum padi, Empoasca spp., Euscelus bilobatus, Nephotettix cincticeps, Lecanium corni, Saissetia oleae, Laodelphax striatellus, Nilaparvata lugens, Aonidiella auroantii, Aspidiotus hederae, Pseudococcus spp., Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Buceulatrix thurberiella, Phyllocnistis citrella, Agrotis spp, Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana. Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochlearieae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa. Aus der Ordnung der Siphonaptera z.B. Xenopsylla cheopis, Ceratophyllus spp.

Aus der Ordnung der Arachnida z.B. Scorpio maurus, Latrodectus mactans.

Gegenstand der Erfindung sind auch Mittel, die die Verbindungen der Formel I neben geeigneten Formulierungshilfsmitteln enthalten.

Die erfindungsgemäßen Mittel enthalten die Wirkstoffe der Formel I, im allgemeinen zu 1 -95 Gew.-%. Sie können als Spritzpulver, emulgierbare Konzentrate, versprühbare Lösungen, Stäubemittel oder Granulate in den üblichen Zubereitungen angewendet werden.

Spritzpulver sind in Wasser gleichmäßig dispergierbare Präparate, die neben dem Wirkstoff außer einem Verdünnungs-oder Inertstoff noch Netzmittel, z.B. polyoxethylierte Alkylphenole, polyoxethylierte Fettalkohole, Alkyl-oder Alkylphenol-sulfonate und Dispergiermittel, z.B. ligninsulfonsaures Natrium, 2,2'-dinaphthylmethan-6,6'-disulfonsaures Natrium, dibutylnapthalinsulfonsaures Natrium oder auch oleylmethyltaurinsaures Natrium enthalten.

Emulgierbare Konzentrate werden durch Auflösen des Wirkstoffes in einem organischen Lösungsmittel, z.B. Butanol, Cyclohexanon, Dimethylformamid, Xylol oder auch höhersiedenden Aromaten oder Kohlenwasserstoffen unter Zusatz von einem oder mehreren Emulgatoren hergestellt. Als Emulgatoren können beispielsweise verwandt werden: Alkylarylsulfonsaure Calzium-Salze wie Cadodecylbenzolsulfonat oder nichtionische Emulgatoren wie Fettsäurepolyglykolester, Alkylarylpolyglykolether, Fettalkoholpolyglykolether,

Propylenoxid-Ethylenoxid-Kondensationsprodukte, Alkylpolyether, Sorbitanfettsäureester, Polyoxyethlensorbitan-Fettsäureester oder Polyoxethylensorbitester.

Stäubemittel erhält man durch Vermahlen des Wirkstoffes mit fein verteilten festen Stoffen, z.B. Talkum, natürlichen Tonen wie Kaolin, Bentonit, Poryphillit oder Diatomeenerde. Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen auf die Oberfläche von Trägerstoffen wie Sand, Kaolinite, oder von granuliertem Inertmaterial. Auch können geeignete Wirkstoffe in der für die Herstellung von Düngemittelgranulaten üblichen Weise -gewünschtenfalls in Mischung mit Düngemitteln -hergestellt werden.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, Formamidine, Zinnverbindungen, durch Mikroorganismen hergestellte Stoffe u.a. Bevorzugte Mischungspartner sind

1. aus der Gruppe der Phosphorverbindungen

Acephate, Azamethiphos, Azinphos-ethyl, Azinphosmethyl, Bromophos, Bromophosethyl, Chlorfenvinphos, Chlormephos, 1-(4-Clorphenyl-4-(O-ethyl,S-propyl)-phosphoryloxypyrazol (TIA 230), Chlorpyrifos, Chlorpyriphos-methyl, Demeton, Demeton-S-methyl, Demeton-S-methyl sulphone, Dialifos, Diazinon, Dichlorvos, Dicrotophos, Dimethoate, Disulfoton, EPN, Ethion, Ethoprophos, Etrimfos, Famphur, Fenamiphos, Fenitrothion, Fensulfothion, Fenthion, Fonofos, Formothion, Heptenophos, Isazophos, Isofenphos, Isothioate, Isoxathion, Malathion, Mephosfolan, Methacrifos, Methamidophos, Methidathion, Salithion, Mevinphos, Monocrotophos, Naled, Omethoate, Oxydemeton-methyl, Parathion, Parathionmethyl, Phenthoate Phorate, Phosalone, Phosfolan, Phosmet, Phosphamidon, Phoxim, Pirimiphos-ethyl, Piriphos-methyl, Profenofos, Propaphos, Propetamphos, Prothiofos, Pyridapenthion, Quinalphos, Sulprofos, Temephos, Terbufos, Tetraclorvinphos, Thiometon, Triazophos, Trichlorphon, Vamidothion;

2. aus der Gruppe der Carbamate

Aldicarb, 2-sec-Butylphenylmethylcarbamate (BPMC), Carbaryl, Carbofuran, Carbosulfan, Cloethocarb, 2,3-Dihydro-2,2-dimethyl-7-benzofuranyl-N-(isoprpyl)-N-(carboethoxyethyl)amino)thiomethylcarbamat (OK-174), Ethiofencarb, Furathiocarb, Isoprocarb, Methomyl, 5-Methyl-m-cumenylbutyryl-(methyl)carbamate, Oxamyl, Piromicarb, Propoxur, Thiocarb, Thifanox, Ethyl 4,6,9-triaza-4-benzyl-6,10-dimethyl-8-oxa-7-oxo-5,11-dithia-9-dodecenoate (OK 135), 1-Methylthio-(ethylideneamino) N-methyl-N-(morpholinothio)carbamate (UC 51717);

3. aus der Gruppe der Carbonsäureester

Allethrin, Alphametrin, 5-Benzyl-3-furylmethyl(E)-(1R)-cis-2,2-dimethyl-3-(2-oxothiolan-3-ylidenemethyl)cyclopropanecarboxylate, Bioallethrin, Bioallethrin((S)-cyclopentylisomer), Bioresmethrin, Biphenate (FMC 54800),α-Cyano-3'-phenoxy-benzyl-2-)4-ethoxyphenyl)-2,2-dichlorocyclopyranecarboxylate (NK 8116, GH 414), (RS)-1-Cyano-1-(6-phenoxy-2-pyridyl)methyl(1RS)-trans-3-(4-tert.butylphenyl)-2,2-dimethylcyclopropanecarboxylate (NCI 85193), Cyfluthrin, Cyhalothrin, Cypermethrin, Deltamethrin, Empenthrin, Esfenvalerate, 5-4-(4-Ethoxyphenyl)-4-methylpentyl)-2-fluoro-1,3-diphenylether (MTI 800), 3-(2-(4-Ethoxyphenyl-2-methylpropoxymethyl)-1,3-diphenylether (MTI 500), Fenfluthrin, Fenpropathrin, Fenvalerate,Flucythrinate, Flumethrin, Fluralinate (Disomers), Permethrin, Phenothrin ((R)-isomers), Pyrethrins (natural products), Resmethrin, Tetramethrin, Tralomethrin;

4. aus der Gruppe der Amidine

Amitraz, Chlordimeform;

5. aus der Gruppe der Zinnverbindungen

Cyhexatin, Fenbutatin oxide;

6. Sonstige

Abamectin, Bacillus thuringensis, Bensultap, Binapacryl, Bromopropylate, Buprofezin, Camphechlor, Cartap, Chlorbenzialate, Chlorfluazuron, 2-(4-Chlorphenyl)-4,5-diphenylthiophen (UBI-T 930), Clofentezine, Cyclopropancarbonsäure(2-napthylmethyl)ester (Ro 12-0470), Cyromazin, DDT, Dicofol, N-(3,5-Dichloro-2,4-difluorphenyl)amino)carbonyl)--2,6-difluorbenzamid (CME 134), N-(3,5-Dichlor-4-(1,1,2,2-tetrafluoroethoxy)phenyl)-amino)carbonyl)-2,6-difluorbenzamide (XRD 473), Diflubenzuron, N-(2,3-Dihydro-3-methyl-1,3-thiazol-2-ylidene)2,4-xylidine, Dinobuton, Dinocap, Endosulfan, Fenoxycarb, Fenthiocarb, Flubenzimine, Gamma-HCH, Hexythiazox, Ivermectin, 2-Nitromethyl-4,5-dihydro-6H-thiazin (SD 52618), 2-Nitromethyl-3,4-dihydrothiazol (SD 35651), Propargite, Tetradifon, Tetrahydro-5,5-dimethyl-2(1H)-pyrimidinone(3-(4-trifluormethyl)phenyl)-1-(2-(4-trifluormethyl)-phenyl)ethenyl-2-propylidene)hydrazone (AC 217300), Tetrasul, Thiocyclam, Triflumuron, Kernpolyeder-und Granuloseviren.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,00001 und 1 Gew.-% liegen.

Die Anwendung geschieht in einer den Anwendungsformen angepaßten üblichen Weise.

Die erfindungsgemäßen Wirkstoffe eignen sich auch zur Bekämpfung von Ekto-und Endoparasiten vorzugsweise von ektoparasitierenden Insekten auf dem veterinärmedizinischen Gebiet bzw. auf dem Gebiet der Tierhaltung.

Die Anwendung der erfindungsgemäßen Wirkstoffe geschieht hier in bekannter Weise, wie durch orale Anwendung in Form von beispielsweise Tabletten, Kapseln, Tränken, Granulaten, durch dermale Anwendung in Form beispielsweise des Tauchens (Dippen), Sprühens (Sprayen), Auf gießens - (pour-on and spot-on) und des Einpuderns sowie durch parenterale Anwendung in Form beispielsweise der Injektion.

Die erfindungsgemäßen neuen Verbindungen der Formel I können demgemäß auch besonders vorteilhaft in der Viehhaltung (z.B. Rinder, Schafe, Schweine und Geflügel wie Hühner, Gänse usw.) eingesetzt werden. In einer bevorzugten Ausführungsform der Erfindung werden den Tieren die neuen Verbindungen, gegebenenfalls in geeigneten Formulierungen (vgl. oben) und gegebenenfalls mit dem Trinkwasser oder Futter oral verabreicht. Da eine Ausscheidung im Kot in wirksamer Weise erfolgt, läßt sich auf diese Weise sehr einfach die Entwicklung von Insekten im Kot der Tiere verhindern. Die jeweils geeigneten Dosierungen und Formulierungen sind insbesondere von der Art und dem Entwicklungsstadium der Nutztiere und auch vom Befallsdruck der Insekten abhängig und lassen sich nach den üblichen Methoden leicht ermitteln und festlegen. Die neuen Verbindungen können bei Rindern z.B. in Dosiermengen von 0,01 bis 1 mg/kg Körpergewicht eingesetzt werden.

Nachfolgende Beispiele dienen zur Erläuterung der Erfindung.

## A. Formulierungsbeispiele

a) Ein Stäubemittel wird erhalten, indem man 10 Gew.-Teile Wirkstoff und 90 Gew.-Teile Talkum als Inertstoff mischt und in einer Schlagmühle zerkleinert.

b) Ein in Wasser leicht dispergierbares, benetzbares Pulver wird erhalten, indem man 25 Gew.-Teile Wirkstoff, 65 Gew.-Teile kaolinhaltigen Quarz als Inertstoff, 10 Gew.-Teile ligninsulfonsaures Kalium und 1 Gew.-Teil oleoylmethyltaurinsaures Natrium als Netz- und Dispergiermittel mischt und in einer Stiftmühle mahlt.

c) Ein in Wasser leicht dispergierbares Dispersionskonzentrat stellt man her, indem man 20 Gew.-Teile Wirkstoff mit 6 Gew.-Teilen Alkyphenolpolyglykolether (Triton X 207), 3 Gew.-Teilen Isotridecanolpolyglykolether (8 AeO) und 72 Gew.-Teilen paraffinischem Mineralöl (Siedebereich z.B. ca. 255 bis über 377°C) mischt und in einer Reibkugelmühle auf eine Feinheit von unter 5 Mikron vermahlt.

d) Ein emulgierbares Konzentrat läßt sich herstellen aus 15 Gew.-Teilen Wirkstoff, 75 Gew.-Teilen Cyclohexanon als Lösungsmittel und 10 Gew.-Teilen oxethyliertes Nonylphenol (10 AeO) als Emulgator.

e) Ein Granulat läßt sich herstellen aus 2 bis 15 Gew.-Teilen Wirkstoff und einem inerten Granulatträgermaterial wie Attapulgit, Bimsgranulat und/oder Quarzsand.

## B. Chemische Beispiele

Beispiel 1

1,87 g (10 mmol) 2,6-Difluorbenzcarboximidsäureethylester in 5 ml absolutem n-Hexan werden bei Raumtemperatur mit 2,24 g (10 mmol) 3,5-Dichlor-2,4-difluorphenylisocyanat versetzt und das Gemisch bei Raumtemperatur gerührt. Der ausgefallene Feststoff wird abgesaugt und aus n-Hexan umkristallisiert. Ausbeute 3,8 g (92%); Fp. 115 -116°C. Analog lassen sich die folgenden Verbindungen der Formel I herstellen.

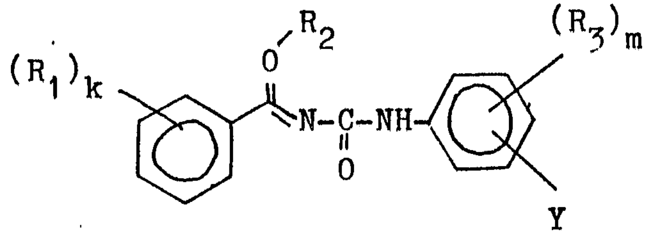

I

Tabelle 1

| Beispiel Nr. | $(R_1)_k$ | $-R^2$ | $(R^3)_m$ | Y | Fp. °C (E/Z) |
|---|---|---|---|---|---|
| 2 | 2,6-$(F)_2$ | $-CH_3$ | 3,5-$Cl_2$,2-F | 4-F | 130 - 132 |
| 3 | " | $-CH_2CCl_3$ | " | " | |
| 4 | " | $-CH_2CF_3$ | " | " | 134 - 135 |
| 5 | " | $-(CH_2)_2CH_3$ | " | " | 98 - 99 |
| 6 | " | $-CH(CH_3)_2$ | " | " | 80 - 81 |
| 7 | " | $-(CH_2)_3CH_3$ | " | " | 120 - 121 |
| 8 | " | $-(CH_2)_5CH_3$ | " | " | 79 - 81 |
| 9 | " | 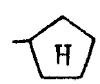 | " | " | |
| 10 | " | 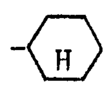 | " | " | 133 - 135 |

Forts. Tabelle 1

| Beispiel Nr. | $(R_1)_k$ | $-R^2$ | $(R^3)_m$ | Y | Fp. °C (E/Z) |
|---|---|---|---|---|---|
| 11 | 2,6-$F_2$ | $-CH_2CH=CH_2$ | " | 4-F | |
| 12 | " | $-CH_2CH=CH-Cl$ | " | " | |
| 13 | " | $-CH_2C≡CH$ | " | " | |
| 14 | " | $-CH_2C≡C-Cl$ | " | " | |
| 15 | " | $-CH_2-C_6H_5$ | " | " | 156 - 158 |
| 16 | " | $-CH_2$- 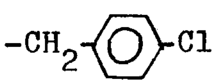 -Cl | " | " | |
| 17 | 2-Cl | $-CH_2CH_3$ | " | 4-(1-pyrrolyl) | 164 - 166 |
| 18 | 2-F | " | " | " | 272 - 174 |
| 19 | 2-Cl, 6-F | " | " | " | |
| 20 | 2,4-$(Cl)_2$ | " | " | " | 258 - 159 |
| 21 | 2,6-$(Cl)_2$ | " | " | " | |

Forts. Tabelle 1.

| Beispiel Nr. | $(R_1)_k$ | $-R^2$ | $(R^3)_m$ | Y | Fp. °C (E/Z) |
|---|---|---|---|---|---|
| 22 | $2,6-(F)_2$ | $-CH_3$ | $3,5-Cl_2$ | 4-(1-pyrroyl) | 149 - 151 |
| 23 | " | $-CH_2CH_3$ | " | " | 153 - 156 |
| 24 | " | $-CH(CH_3)_2$ | " | " | |
| 25 | $2-CH_3$ | $-CH_2CH_3$ | " | " | |
| 26 | $2-CF_3$ | " | " | " | |
| 27 | $2-OCH_3$ | " | " | " | |
| 28 | $2-OCHF_2$ | " | " | " | |
| 29 | $2,4,6-F_3$ | " | " | " | |
| 30 | $2,6-F_2$ | " | 3-Cl | " | 125 - 128 |
| 31 | $2,6-F_2$ | " | H | " | 116 - 118 |
| 32 | 2-Cl | " | H | 3-(1-pyrrolyl) | |
| 33 | 2-F | " | H | " | |

Forts. Tabelle 1

| Beispiel Nr. | $(R_1)_k$ | $-R^2$ | $(R^3)_m$ | Y | Fp. °C (E/Z) |
|---|---|---|---|---|---|
| 34 | 2,6-$F_2$ | $-CH_2CH_3$ | H | 3-(1-pyrrolyl) | |
| 35 | " | " | H | 3-(4-$CF_3O$-$C_6H_4$-O) | |
| 36 | " | " | 4-Cl | " | |
| 37 | 2-F | " | 3,5-$(Cl)_2$ | 4-(4-$CF_2HO$-$C_6H_4$-O) | 108 - 109 |
| 38 | 2-Cl | " | " | " | |
| 39 | 2,6-$F_2$ | " | " | " | 111 - 112 |
| 40 | 2-F | " | " | 4-(4-$CF_3O$-$C_6H_4O$) | 110 - 111 |
| 41 | 2-Cl | " | " | " | 115 - 118 |
| 42 | 2,6-$F_2$ | " | " | " | 102 - 104 |
| 43 | 2-F | " | " | 4-(3-Cl,4-$CF_3O$-$C_6H_3$-O) | |
| 44 | 2-Cl | " | " | " | |

Forts. Tabelle 1

| Beispiel Nr. | $(R_1)_k$ | $-R^2$ | $(R^3)_m$ | Y | Fp. °C (E/Z) |
|---|---|---|---|---|---|
| 45 | 2,6-$F_2$ | $-CH_2CH_3$ | 3,5-$(Cl)_2$ | 4-(3-Cl,4-$CF_3O$-$C_6H_3$-O) | 114 - 116 |
| 46 | 2-Cl | " | " | 4-[4-($CF_2H$-$CF_2O$)-$C_6H_4$-O] | 126 - 129 |
| 47 | 2-F | " | " | " | 111 - 113 |
| 48 | 2-Cl, 6-F | " | " | " | 112 - 114 |
| 49 | 2,4-$Cl_2$ | " | " | " | |
| 50 | 2,6-$Cl_2$ | " | " | " | |
| 51 | 2,6-$F_2$ | " | " | " | 94 - 96 |
| 52 | 2-$CH_3$ | " | " | " | |
| 53 | 2-$CF_3$ | " | " | " | 107 - 109 |
| 54 | 2-$OCH_3$ | " | " | " | 116 - 119 |
| 55 | 2-$OCHF_2$ | " | " | " | 94 - 96 |

Forts. Tabelle 1

| Beispiel Nr. | $(R_1)_k$ | $-R^2$ | $(R^3)_m$ | Y | Fp. °C (E/Z) |
|---|---|---|---|---|---|
| 56 | 2,6-$F_2$ | $-CH_2CH_3$ | 3-Cl, 5-$CH_3$ | 4-[4-$CF_2H$-$CF_2O$)-$C_6H_4$-O] | |
| 57 | " | " | 3-Cl, 5-$OCH_3$ | " | |
| 58 | " | " | 3-Cl,5-$CF_3$ | " | |
| 59 | 2-Cl | " | 3,5-$Cl_2$ | 4-[3-Cl,4-($CF_2H$-$CF_2O$)-$C_6H_3$-O] | 130 - 131 |
| 60 | 2-F | " | " | " | 121 - 122 |
| 61 | 2,6-$(F)_2$ | " | " | " | 104 - 107 |
| 62 | 2-Cl | " | " | 4-[2-Cl,4-($CF_2H$-$CF_2O$)-$C_6H_3$-O] | |
| 63 | 2-F | " | " | " | |
| 64 | 2;6-$F_2$ | " | " | " | 103 - 105 |
| 65 | 2-Cl | " | " | 4-[2,6-$Cl_2$,4-($CF_2H$-$CF_2O$)-$C_6H_2$-O] | |

Forts. Tabelle 1

| Beispiel Nr. | $(R_1)_k$ | $-R^2$ | $(R^3)_m$ | Y | Fp. °C (E/Z) |
|---|---|---|---|---|---|
| 66 | 2-F | $-CH_2CH_3$ | 3,5-$(Cl)_2$ | 4-[2,6-$Cl_2$,4($CF_2H$-$CF_2O$)-$C_6H_2$-O] | |
| 67 | 2,6-$(F)_2$ | " | " | " | 108 - 110 |
| 68 | 2-Cl | " | " | " | |
| 69 | 2-F | " | " | " | |
| 70 | 2,6-$(F)_2$ | " | " | " | |
| 71 | 2-$CH_3$ | " | " | " | |
| 72 | 2,6-$(F)_2$ | " | 2,3,5-$Cl_3$ | " | |
| 73 | " | " | 3,5-$Cl_2$,2-F | " | |
| 74 | 2-Cl | " | 3,5-$Cl_2$ | 4-[3-Cl,4-($CF_3$-CHF-$CF_2O$)-$C_6H_3$-O] | |
| 75 | 2-F | " | " | " | |
| 76 | 2,6-$F_2$ | " | " | " | |

Forts. Tabelle 1

| Beispiel Nr. | $(R_1)_k$ | $-R^2$ | $(R^3)_m$ | Y | Fp. °C (E/Z) |
|---|---|---|---|---|---|
| 77 | 2-Cl | $-CH_2CH_3$ | 3,5-$(Cl)_2$ | 4-[2-Cl,4-($CF_3$-CHF-$CF_2O$)-$C_6H_3$-O] | 131 - 133 |
| 78 | 2,6-$(F)_2$ | " | " | " | 126 - 128 |
| 79 | 2-Cl | " | " | 4-[2,6-$Cl_2$,4-($CF_3$CHF-$CF_2O$)-$C_6H_2$-O] | |
| 80 | 2-F | " | " | " | |
| 81 | 2,6-$(F)_2$ | " | " | " | 133 - 135 |
| 82 | " | " | " | 4-[3-$CF_3$,4-($CF_3$-CHF-$CF_2O$)-$C_6H_3$-O] | |
| 83 | " | " | " | 4-[3,4$(CF_2HO)_2$-$C_6H_3$-O] | |

C. Biologische Beispiele

Beispiel I

Spodoptera-Test

Larven des afrikanischen Baumwollwurms - (Spodoptera littoralis L III) und Petrischalen, in die eine Diät auf Agar-Basis eingefüllt wird, wurden in einer Spritzapparatur mit einer Wirkstoffzubereitung der gewünschten Konzentration behandelt. Die Larven wurden nach Antrocknen des Spriztbelages auf die Agar-Diät gesetzt.

Nach der gewünschten Zeit (L III bis Falterschlupf) wurde die Abtötung der Raupen bzw. der Schlupf der Falter in % bestimmt. Dabei bedeutet 100 %, daß alle Raupen abgetötet wurden bzw. kein Falter aus den Raupen schlüpfte.

Bei diesem Test zeigten bei einem Versuch mit einer Wirkstoffkonzentration von 0,01 % die Verbindungen 1, 18, 46, 47 und 51 einen Wirkungsgrad von 100 %.

Beispiel II

Musca-Test

24 Stunden alte Hausfliegenlarven (Musca domestica) wurden in eine Fliegendiät, die vorher mit einer Wirkstoffzubereitung der gewünschten Konzentration behandelt wurde, eingebracht.

Nach der gewünschten Zeit (L I bis Fliegenschlupf) wurde die Abtötung der Larven bzw. der Schlupf der Fliegen in % bestimmt. Dabei bedeutet 100 %, daß alle Larven abgetötet wurden bzw. keine Fliege aus den Puppen schlüpfte.

Bei diesem Test zeigten bei einer Wirkstoffkonzentration von 0,01 % die Verbindungen 1, 17, 18, 23, 46, 47, 48 und 51 einen Wirkungsgrad von 80 - 100 %.

Beispiel III

Aedes-Test

Man füllte die wäßrigen Wirkstoffzubereitungen der gewünschten Konzentration in Erlenmeyerkolben und setzte anschließend 24 Stunden alte Gelbfiebermückenlarven (Aedes ägypti) in die Kolben.

Nach der gewünschten Zeit (bis zum Mückenschlupf) wurde die Abtötung der Larven bzw. der Schlupf der Mücken in % bestimmt. Dabei bedeutet 100 %, daß alle Larven abgetötet wurden bzw. keine Mücke schlüpfte.

Bei diesem Test zeigten die Verbindungen 1, 18, 23 und 51 bei einer Wirkstoffkonzentration von 0,001 % einen Wirkungsgrad von 100 %.

Beispiel IV

Epilachna-Test

Larven des mexikanischen Bohnenkäfers - (Epilachna varivestis L III) wurden in einer Spritzapparatur mit einer Wirkstoffzubereitung der gewünschten Konzentration behandelt. Gleichzeitig wurden Buschbohnenblätter (Phaseolus vulgaris) in die entsprechende Wirkstofflösung getaucht. Nach dem Antrocknen des Spritzbelages wurden die Larven des Käfers auf die Bohnenblätter gesetzt.

Nach der gewünschten Zeit (L III bis Käferschlupf) wurde die Abtötung der Larven bzw. der Schlupf der Käfer in % bestimmt. Dabei bedeutet 100 %, daß alle Larven abgetötet wurden bzw. kein Käfer aus den Puppen schlüpfte.

Bei diesem Test zeigte die Verbindung 1 bei einer Wirkstoffkonzentration von 0,1 % einen Wirkungsgrad von 100 %.

Beispiel V

Oncopeltus-Test

Larven einer Baumwollwanze (Oncopeltus fasciatus L III) wurden zusammen mit einem Dentalröllchen, das vorher mit einer Wirkstoffzubereitung der gewünschten Konzentration behandelt wurde, in einen Plastikbecher gegeben.

Nach der gewünschten Zeit (L III bis Imago) wurde die Abtötung der Larven bzw. der Schlupf der Imago in % bestimmt. Dabei bedeutet 100 %, daß alle Larven abgetötet wurden bzw. keine Imago aus dem letzten Larvenstadium schlüpfte.

Bei diesem Test zeigte die Verbindung 1 bei einer Wirkstoffkonzentration von 0,1 % einen Wirkungsgrad von 100 %.

Beispiel VI: Wirkung gegen Lucilia cuprina

Die Aktivsubstanzen werden in einer Mischung bestehend aus Dimethylformamid (85 g), $^{R}$Emulsogen (7 g) und $^{R}$Arkopal N 60 (3 g) so gelöst, daß Verdünnungsreihen mit Wirkstoffkonzentrationen von 10000/1000/100/10 ppm erhalten werden. Jeweils 1 ml dieser Lösungen wird mit 9 g fein gemahlenem Fleisch innig vermischt, so daß letztlich Fleisch mit Wirkstoffkonzentrationen von 1000/100/10/1 ppm vorhanden ist.

Zur Kontrolle werden 9 g Fleisch mit 1 ml des Lösungsmittels versetzt.

Den so präparierten Larvennährmedien werden jeweils 20 frisch geschlüpfte Larven I von Lucilia cuprina zugegeben.

Nach 72 Stunden, wenn sich im Kontrollmedium die Larven I zu verpuppungsreifen Larven III entwickelt haben, wird die Mortalitätsrate ermittelt.

In diesem Test erbrachte die Verbindung Nr. 23 bei einer Wirkstoffkonzentration von 10 ppm 100 % Abtötung der Larven.

**Ansprüche**

1. Verbindungen der Formel I

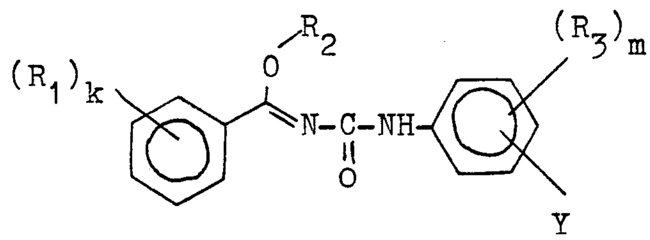

(I),

worin Y= einen Rest 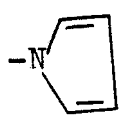 , 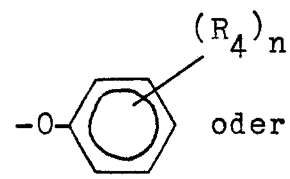 oder 4-Fluor

$R_1$ = Wasserstoff, Halogen, $(C_1$-$C_4)$Alkyl, $(C_1$-$C_4)$-Alkoxy, die beide ein-oder mehrfach durch Halogen substituiert sein können,

$R_2$ = $(C_1$-$C_6)$Alkyl, $(C_3$-$C_6)$Cycloalkyl, $(C_3$-$C_6)$Alkenyl, $(C_3$-$C_6)$Alkinyl oder Benzyl, wobei alle diese Reste ein-oder mehrfach durch Halogen substituiert sein können,

$R_3$, $R_4$ = unabhängig voneinander Wasserstoff, Halogen, $(C_1$-$C_6)$Alkyl, $(C_1$-$C_6)$Alkoxy, die beide ein- oder mehrfach durch Halogen substituiert sein können,

k, m, n = unabhängig voneinander eine ganze Zahl von 1 bis 3 bedeuten, mit der Maßgabe, daß, wenn Y = 4-Fluor bedeutet, $(R_3)_m$ für 3,5-Dichlor-2-fluor und $(R_1)_k$ für 2,6-Difluor stehen muß.

2. Verfahren zur Herstellung von Verbindungen der Formel I, dadurch gekennzeichnet, daß man

a) einen substituierten Arylcarboximidsäureester der Formel (II)

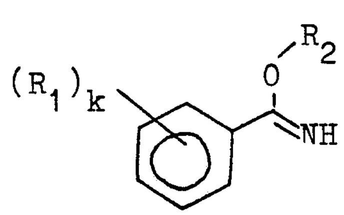

(II)

mit einer Verbindung der Formel (III)

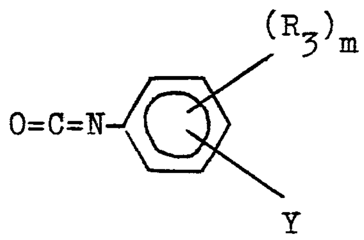

(III)

oder mit einer Verbindung der Formel (IV)

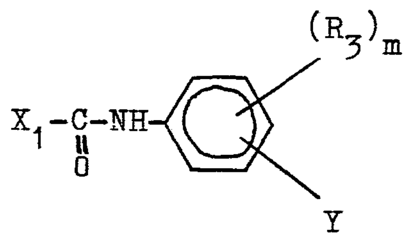

(IV)

worin $X_1$ eine nucleofuge Abgangsgruppe außer Halogen bedeutet, oder

b) eine substituiertes Arylcarboximidsäureester-Derivat der Formel (V)

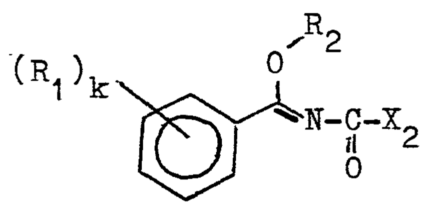

(V)

worin $X_2$ die Bedeutung von $X_1$ besitzt und zusätzlich für Halogen steht,

mit einem substituierten Anilin der Formel (VI)

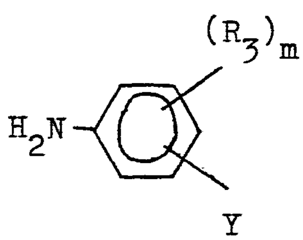

(VI)

c) ein substituiertes 1,3,5-Oxadiazin der Formel - (VII)

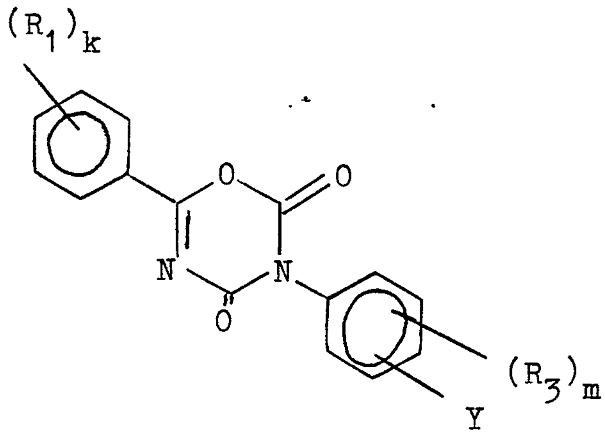

mit einem Alkohol der Formel (VIII)

$R_2$-OH (VIII)

umsetzt.

3. Insektizide Mittel, dadurch gekennzeichnet, daß sie eine Verbindung der Formel I von Anspruch 1 als Wirkstoff und Formulierungshilfsmittel enthalten.

4. Verwendung von Verbindungen der Formel I in Anspruch 1 zur Bekämpfung von Schadinsekten.

5. Verfahren zur Bekämpfung von Schadinsekten, dadurch gekennzeichnet, daß man auf diese, die von ihnen befallenen Pflanzen oder Anbauflächen eine wirksame Menge einer Verbindung der Formel I von Anspruch 1 aufbringt.

Patentansprüche für den Vertragsstaat Österreich

1. Verfahren zur Herstellung der Verbindungen der Formel I

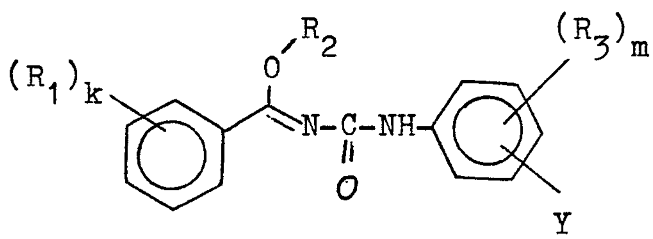

(I)

worin Y= einen Rest 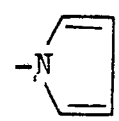 , 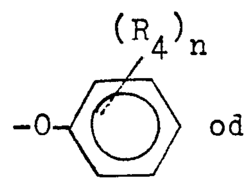 oder 4-Fluor

$R_1$ = Wasserstoff, Halogen, $(C_1$-$C_4)$Alkyl, $(C_1$-$C_4)$-Alkoxy, die beide ein-oder mehrfach durch Halogen substituiert sein können,

$R_2$ = $(C_1$-$C_6)$Alkyl, $(C_3$-$C_6)$Cycloalkyl, $(C_3$-$C_6)$Alkenyl, $(C_3$-$C_6)$Alkinyl oder Benzyl, wobei alle diese Reste ein-oder mehrfach durch Halogen substituiert sein können,

$R_3$, $R_4$ = unabhängig voneinander Wasserstoff, Halogen, $(C_1$-$C_6)$Alkyl, $(C_1$-$C_6)$Alkoxy, die beide ein- oder mehrfach durch Halogen substituiert sein können,

k, m, n = unabhängig voneinander eine ganze Zahl von 1 bis 3 bedeuten, mit der Maßgabe, daß wenn Y = 4-Fluor bedeutet, $(R_3)_m$ für 3,5-Dichlor-2-fluor und $(R_1)_k$ für 2,6-Difluor stehen muß,

dadurch gekennzeichnet, daß man

a) einen substituierten Arylcarboximidsäureester der Formel (II)

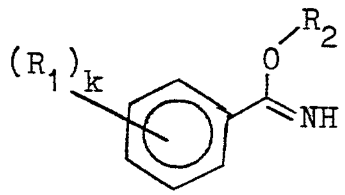

(II)

mit einer Verbindung der Formel (III)

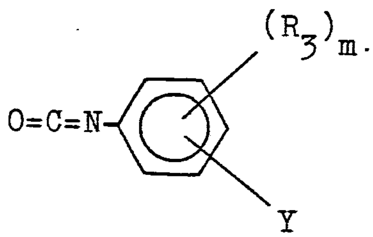

(III)

oder mit einer Verbindung der Formel (IV)

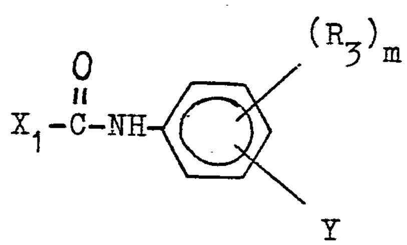

(IV)

worin $X_1$ eine nucleofuge Abgangsgruppe außer Halogen bedeutet, oder

b) ein substituiertes Arylcarboximidsäureester-Derivat der Formel (V)

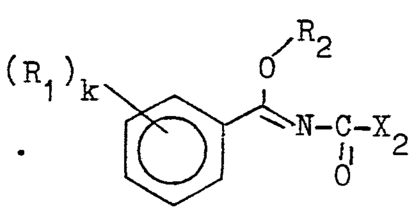

(V)

worin $X_2$ die Bedeutung von $X_1$ besitzt und zusätzlich für Halogen steht,

mit einem substituierten Anilin der Formel (VI)

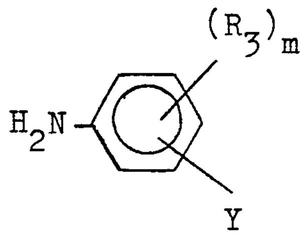

(VI)

c) ein substituiertes 1,3,5-Oxadiazin der Formel - (VII)

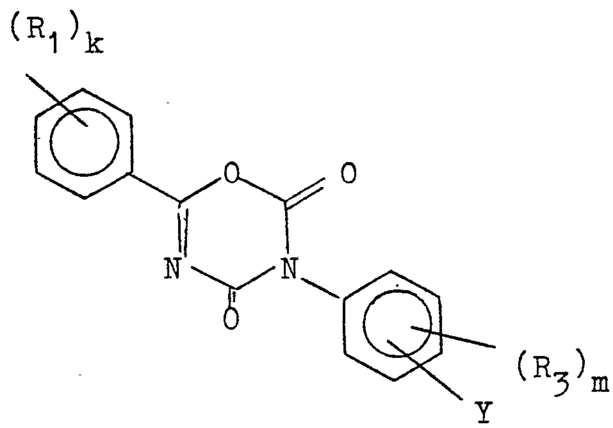

mit einem Alkohol der Formel (VIII)

$R_2$-OH (VIII)

umsetzt.

2. Insektizide Mittel, dadurch gekennzeichnet, daß sie eine Verbindung der Formel I von Anspruch 1 als Wirkstoff und Formulierungshilfsmittel enthalten.

3. Verwendung von Verbindungen der Formel I in Anspruch 1 zur Bekämpfung von Schadinsekten.

4. Verfahren zur Bekämpfung von Schadinsekten, dadurch gekennzeichnet, daß man auf diese, die von ihnen befallenen Pflanzen oder Anbauflächen eine wirksame Menge einer Verbindung der Formel I von Anspruch 1 aufbringt.